# EUROPEAN PATENT SPECIFICATION

(11) **EP 0 371 017 B1**
(45) Date of publication and mention of the grant of the patent: **21.09.1994**
(21) Application number: 88901890.9
(22) Date of filing: 26.02.1988
(51) Int. Cl.: A61K 39/40, C12P 21/00, A61K 7/16

(54) **ANTIBODIES AGAINST STREPTOCOCCUS**
ANTIKÖRPER GEGEN STREPTOCOCCUS
ANTICORPS DRESSES CONTRE LES STREPTOCOQUES

(30) Priority: 27.02.1987 GB 8704648
(43) Date of publication of application: 06.06.1990
(73) Proprietor: COUNCIL OF GOVERNORS OF THE UNITED MEDICAL AND DENTAL SCHOOLS OF GUY'S AND ST. THOMAS'S HOSPITALS, London, SE1. (GB)
(72) Inventor: LEHNER, Thomas, London SE1 9RT (GB); SMITH, Roberta, London SE1 9RT (GB)
(74) Representative: Goldin, Douglas Michael
(86) International application number: GB8800135
(87) International publication number: WO8806455

(56) References cited:
- EP-A- 0 116 472
- EP-A- 0 140 498
- WO-A-81/01101
- GB-A- 2 167 299
- CHEMICAL ABSTRACTS, vol. 103, no. 17, 28 October 1985, Columbus, OH (US); p. 558, no. 139998v#
- INFECTION & IMMUNITY, vol. 46, no. 1; pp. 168-175#

## Description

### FIELD OF THE INVENTION

THIS INVENTION relates to antibodies useful to combat dental caries.

### BACKGROUND TO THE INVENTION

Streptococcus mutans has been recognised for many years as the major organism responsible for the development of dental caries in mammals. Various vaccines have been proposed in the past based on various antigenic fragments of S. mutans. One such vaccine is described in British Patent No. 2,060,647 based upon the antigen known as I or I/II. Antigen I has a molecular weight, as determined by sodium dodecyl sulphate polyacrylamide gel electrophoresis (SDS-PAGE) of 146-155 Kd. Antigen I/II is believed to be a conjugate of antigen I and antigen II, this I/II antigen having a molecular weight determined by SDS-PAGE of 175-195 Kd. Published European Patent Application No. EP-A-0 116 472 describes antigen X which is a much smaller molecule having a molecular weight, determined by SDS-PAGE of about 3.5-4.5 Kd but which appears to include the same antigenic determinants included within antigens I and I/II.

Antibodies against antigens I, I/II and X are known. The above-mentioned British Patent describes the raising of antibodies against antigens I and I/II by conventional procedures in experimental animals, for example rhesus monkeys, rabbits and mice. These antibodies are proposed primarily for the purification of the antigen by affinity chromatography but the Patent Specification mentions the possibility of using such antibodies for passive immunisation by conventional means. Conventional passive immunisation involves parenteral administration of the antibodies but while such techniques are theoretically available, as a practical matter, passive immunisation has never been regarded as clinically attractive and indeed, the British Patent refers to the preferred use of the antigenic materials for direct immunisation.

GB 2 167 299 and EP 0 140 498 describe an antibody raised using S. mutans as immunogen in association with an alkanolamide fatty acid ester (GB 2 167 299) or in association with an unusually diverse group of synergists (EP 0 140 498). In both cases resides in the formulation of the antibody with a further component presumably supposed to improve the effect of the antibody.

All antibodies that have been raised against S. mutans serotype c or against the streptococcal antigen serotype c (SA_{c}) exhibit a certain degree of cross-reactivity. It is well known that such antibodies are also cross-reactive with antigenic material originating from serotypes e and f of S. mutans. In clinical practice, it is found that serotypes c, e and f amount to about 90% of the bacterial S. mutans population so that the prophylactic or therapeutic use of antibodies raised against serotype c are of considerable practical value but fail to be effective in relation to the residual approximately 10% of the bacterial population. In some series serotype d is found in addition to serotype c in up to 50% of children examined.

This residual 10% is comprised predominantly of a serotype d that until very recently has been regarded merely as another serotype of S. mutans. However recently, this particular serotype has been reclassified as S. sobrinus and, in the description of this invention, we will use the nomenclature S. sobrinus serotype d rather than S. mutans serotype d.

We have now found that if antibodies are raised against S. sobrinus serotype d, that many of the resulting antibodies are cross-reactive not only with S. sobrinus serotype d and also S. sobrinus (S. mutans) serotype g (and serotype a) but surprisingly, that such antibodies are also cross-reactive with serotypes c, e and f of S. mutans. Since somewhere of the order of 98% of all serotypes of S. mutans/S. sobrinus found in the oral cavities are of serotypes a, c, d, e, f and g, our discovery has enabled us to produce, for the first time, antibodies having the potential for prophylactic and/or therapeutic use in relation to substantially 100% of oral bacteria of S. mutans/S.sobrinus type that are responsible for dental caries.

The present invention provides an antibody or fragment thereof, which is able to combat dental caries, said antibody being obtainable by using as immunogen, S. sobrinus serotype d or streptococcal antigen thereof or a fragment of said antigen, said antigen fragment retaining the antigenic determinants characteristic of S. sobrinus serotype d, the antibody or fragment thereof cross reacting with S. mutans serotypes c, e, f and g.

The antibodies of the present invention extend not only to whole antibodies but also to antibody fragments containing the necessary binding sites to enable them to recognise and bind with the streptococcal antigen and the antibodies or fragments thereof may be prepared in polyclonal or monoclonal form. The antibody or fragment thereof may be, for example, of the IgG1 or IgG2b class.

The antibodies of the invention can be raised using S. sobrinus serotype d or streptococcal antigen derived therefrom as the immunogen. Normally, the immunogen will comprise the naturally-occurring streptococcal antigen but, as is the case with the use of streptococcal antigen from serotype c of S. mutans, use can also be made of antigenic fragments of serotype d, provided that those antigenic fragments contain the necessary antigenic determinants characteristic of serotype d.

For the production of polyclonal antibodies, conventional methods may be used involving the immunisation of animals with the serotype d immunogen followed by recovery of the antibodies from the blood of the immunised animals. Conventional antibody recovery methods can be used and conventional antibody purification methods can be used, e.g. affinity chromatography using purified immunogen or fragments thereof.

Where monoclonal antibodies are to be raised, the immunogen derived from serotype d can be used conventionally to immunise mice or other mammals and the spleen of the immunised mammal hybridised with myeloma cells to produce a population of hybridoma cells. Alternatively, immunogen derived from serotype d can be used for in vitro stimulation of B-lymphocytes and the stimulated B-lymphocytes then hybridised with myeloma cells by methods known per se to provide a population of hybridomas. The resulting population of hybridomas may then be screened to select those secreting monoclonal antibody that is cross-reactive by S. sobrinus serotype d and S. mutans serotype c. When polyclonal antibodies are produced, they may also be screened to select those showing the serotype d/c cross-reactivity. The antibodies, however produced, can be purified if necessary by affinity chromatography techniques or by staphylococcal protein A to separate IgG class of antibodies or by using serotype d antigen.

Where antibody fragments are required, the polyclonal or monoclonal antibody produced by the methods described above can be fragmented by digestion with papain or pepsin by conventional methods.

### DETAILED DESCRIPTION OF THE INVENTION

While the present invention is directed primarily to humans, the problem of dental caries is not confined to humans but arises in other mammals including non-human primates, domestic and farm animals and particularly in those cases where the non-human mammal eats a substantial proportion of food including sugars.

The monoclonal antibodies used in this invention may be applied to the tooth in the mouth of the mammal by any convenient method. Numerous methods are now available for the treatment of teeth with various materials for various purposes. If the treatment is to be carried out by a Dental Surgeon, then the monoclonal antibody is conveniently applied by painting the surface of the tooth. If the monoclonal antibodies are to be self-applied, then the monoclonal antibodies may be included in a toothpaste, mouthwash, chewing gum, lozenge or gel. As will be described in more detail below, the duration of protection afforded by the method of the present invention is surprisingly long but the frequency of the topical application is primarily one for the users personal convenience. Methods of self-application from toothpastes etc., can result in applications being repeated perhaps daily while the use of lozenges can result in more frequent application of the antibody. Chewing gums and gels may be regarded, for this purpose, as providing a certain amount of sustained release of the antibody over a period of half-an-hour or more and indeed, if sustained release of the antibody is required, then appropriate formulations can be used that will result in slow release of antibody into the mouth from the formulation as a result of the temperature or saliva conditions etc., found in the mouth. In certain instances, it may be desirable to provide a more formal prolonged contact of the antibody with the tooth surface and in such cases, appropriate dental trays can be used that will cover the tooth after it has been coated with antibody and prevent the antibody from being removed, e.g. by saliva, for a predetermined period.

It is important that the antibody be brought into contact with the surface of the tooth and ideally should be applied to all of the smooth and occlusal surfaces of the tooth. It is not detrimental for the antibody to contact the gum but the protection afforded by the present invention does appear to result primarily if not exclusively from the contact of the antibody with the surface of the tooth itself.

Topical administration of the antibodies is the most practical course for self-administration.

The exact amount of antibody that is applied does not appear to be critical since, in a method of this type, repeated self-application of antibody is not difficult and indeed, particularly after initial treatment by a Dental Surgeon, maintenance or top-up treatment can be carried out by the user at whatever frequency is desirable. By way of guidance, it can be indicated that somewhere of the order of 10 to 100 micrograms of antibody can be applied to each tooth on each occasion that antibody is applied but amounts of antibody outside this range can certainly be applied without causing detriment to the subject. The use of insufficient quantities of antibody simply means that the level of protection is not as great as would otherwise be obtainable while the use of excessive amounts of antibody does not improve the protection and simply results in unnecessary use of antibody.

The exact formulation for the antibody is not a matter of critical importance but depends entirely upon the method of application to be adopted and the convenience of the user. In all cases, it is important to formulate the antibody in an environment of appropriate pH and which is free from other deleterious materials which might bring about protein degradation and the formulation should, of course, also be free from microbial impurity that would be deleterious in the subject's mouth. For example, for use in the dental surgery, the antibody could be formulated as a simple aqueous dispersion containing somewhere in the region of 0.1 to 10 milligrams of antibody per 100 microlitres of liquid and a liquid of such concentration could be applied to the tooth at the rate of about 1 to 10 microlitres of dispersion per tooth. Where the antibody is to be formulated for self-administration, then the concentration can be selected bearing in mind the above guidelines, the quantities of the formulation that are normally taken on each occasion of self-administration and the fact that over administration of antibody will not be deleterious.

The present invention also provides a dental composition, suitable for application to the teeth, comprising an antibody or fragment thereof as defined above together with a carrier or diluent. The present invention further provides an antibody or fragment thereof, or a composition, as defined above, for use in a method of combatting dental caries in a mammel.

The following Examples are given to illustrate the invention.

### Materials and Methods

### Organisms

S. mutans serotypes and strains were obtained from a number of laboratories as described previously (Smith, Lehner, Beverlev, 1984). Samples of serotype d strains (FRID and Lee) were isolated in our laboratories. Strain AHT, originally described as serotype a however, has been apparently reclassified to serotype g; hence no results are presented for serotype a.

### Streptococcal antigens (SA)

SA from serotype d (strains FRID and OMZ 176) were prepared from culture supernatants by combination of ammonium sulphate precipitation, ion exchange chromatography and gel filtration, as described previously (Russell et al 1980). Antigen dI/II, equivalent to cI/II of serotype c (185 Kd) was characterised using cross-reactive polyclonal rabbit antisera to SA cI/II.

### Production of monoclonal antibodies (McAb)

McAb to SA dI/II were prepared by a method similar to that of Smith et al 1984 following the procedure of Fazekas de St. Groth and Scheidegger (1980). Briefly Balb c mice were immunised with 10 ug SA dI/II (strain FRID) intraperitoneally in Freund's complete adjuvant, followed two weeks later by 10 ug SA dI/II in Freund's incomplete adjuvant. The third and final injection, again two weeks later was given intravenously in saline and the mouse was bled and sacrificed four days later and the spleen was removed. Immune mouse spleen cells were fused at a ratio of 1:1 with the mouse myeloma line P3-NS1/1-Ag4-1 (NS1), in the presence of 50% (wt/vol) polyethylene glycol (PEG 4000 d; Sigma chemical Co.) in RPM1 1640 (Gibco Laboratories). The PEG was neutralised with 10N sodium hydroxide prior to use.

After fusion the cells were plated out at 2 x 10⁵ total viable cells per well, in 24 well cluster plates (Costar plastics, Cambridge, Mass.). Feeder cells from peritoneal lavage of BALB/c mice were added at 3 x 10⁴ cell/well. Throughout cloning, RPM1 1640, supplemented with 10% foetal calf serum, glutamine (2mM), pyruvate (1mM) and antibiotics was used. For the first two weeks after fusion the medium was further supplemented with hypoxanthine, aminopterin and thymidine to eliminate unfused NS1 cells. By the third week only hypoxanthine and thymidine were added. After 10 to 14 days the supernatants of wells showing growth were assayed for specific antibodies to the purified SA and to heart homogenate (Bergmeier and Lehner 1983) in a micro-solid-phase radioassay, as described previously (Smith et al 1984). Cultures giving binding greater than 3 times the background were cloned, at least twice by the method of limiting dilution to 0.3 cells per well and dispensed in the presence of 5 x 10³ feeder cells and 5 x 10⁴ normal spleen cells, into 96-well plates, with flat bottom wells (Costar). Cloned cells were expanded and then injected into pristane primed mice for ascites. Culture supernatants were stored at 4^{o}C with 0.1% sodium azide and ascites at -20^{o}C. The cells were frozen in the presence of 10% dimethyl sulphoxide, 50% foetal calf serum in RPM1 1640 and stored in liquid nitrogen.

### Characterisation of the McAb

The isotype of the McAb was determined by double diffusion precipitation in 1% agarose, using culture supernatants of the McAb and reactions with commercial antisera (Nordic) for IgG1, IgG2a and (Eivai Bios) for IgM. The specificity of the McAb was tested by titrating each McAb against purified SA dI/II, derived from S. sobrinus serotype d (FRID, OMZ 176) or S. mutans serotype c (Guy's strain) as well as against whole cells of the other serotypes and S. sanguis (OMZ 9), by the radioassay (Smith et al 1984).

### Inhibition studies of McAb with purified SA for antigen specificity

Ascitic fluids of the McAb were diluted with PBS containing 0.5% bovine serum albumin and 0.05% Tween® 20, to give approximately 50% binding to SA dI/II (FRID) by micro-solid-phase radioassay. 200 µl samples of dilute McAb were incubated with 5 µg of SA dI/II (FRID), or saline overnight at 4^{o}C. The samples were then examined by the radioassay for antibody binding to SA dI/II.

### Results

### Characterisation of McAb

**TABLE 1**

| Code | Strep. mutans antigen specificity | Antibody class | Serotype specificity |
|---|---|---|---|
| Guy's 1 | serotype c I/III | IgG2a | c, e, f |
| Guy's 12 | serotype c I/II & d I/II | IgG1 | c,d,e,f,g |
| Guy's 13 | serotype c I/II & d I/II | IgG1 | a,c,d,e,f,g |
| serotype h not tested | | | |

We have prepared and characterised various monoclonal antibodies (McAb) against various SA from serotype c and d (Table 1). Guy's 1 (formerly 2D4), was prepared using the antigen for which it is specific as immunogen. McAb to SA dI/II, Guy's 12 and 13 were derived from a single fusion, using SA dI/II (FRID) as immunogen. The McAb belong to IgG1 class as shown in Table 1.

Ascitic fluid of the McAb titrate to >10⁻⁶, as measured by the radioassay against SA dI/II for Guy's 12, 13 or SA cI/II for Guy's 1. Similarly, using the fluid phase radioassay against whole cells of S. mutans, the McAb which bind to SA dI/II also bind in high titres (>10⁻⁶) to the cell surface of serotype d (OMZ 176). Guy's 1, equivalent to SA cI/II, also shows titres of >10⁻⁶ against the whole cells of serotype c (Guy's).

### SA specificity

The specificity of McAb Guy's 1 has been demonstrated previously for SA cI/II, I, II and III by direct binding and competitive inhibition in the radioassay. Examination of the binding of Guy's 1 to SA dI/II was negative. However, Guy's 12 and 13 react with SA I/II derived from both S. mutans and S. sobrinus.

### Serotype specificity

Binding of McAb to the surface of different serotypes of S. mutans and S. sobrinus was examined by the fluid phase radioassay against whole cells. Guy's 1 bound to the surface of serotype c (Guy's strain) and cross-reacted with serotype f (OMZ 175). It also showed low but significant binding to serotype e, giving the classical serotype cross-reactions for S. mutans (c, e, f). Guy's 12 and 13 reacted with S. mutans serotypes, c, e, f and a (except 12) and S. sobrinus serotypes d and g. None of the McAb gave any significant binding to S. sanguis.

We have prepared McAb which are specific for S. mutans serotypes c, e and f and S. sobrinus serotypes d and g. McAb to S. mutans or S. sobrinus are of the IgG1 and IgG2a classes but S. sobrinus also elicited McAb of the IgM class. We have demonstrated the specificity of McAb for cell surface SA I/II, I, II or III by direct binding and competitive inhibition. McAb reacting to serotype cI/II do not bind to serotype dI/II but the three monoclonal antibodies raised by immunisation with S. sobrinus showed a broad reactivity with S. mutans and S. sobrinus serotypes. This suggests that Guy's 12 and 13 are most likely directed against a common determinant of the mutans type of streptococci and are therefore particularly convenient reagents to be used against colonisation of S. mutans and S. sobrinus. These SA are expressed on the cell surface (Zanders and Lehner 1981, Moro and Russell 1985) and are here shown to bind strongly McAb in a fluid phase radioassay. The McAb show high titres (>10⁻⁶), in binding both to purified SA and to the SA on the cell surface.

### REFERENCES

1. Bergmeier L.A. and T. Lehner. 1983. Infect. Immun. 40:1075-1082.
2. Fazekas de St. Groth S. and D. Scheidegger. 1980. J. Immunol. Methods 35:1-21.
3. Moro I. and M.W. Russell. 1983. Infect. Immun. 41:410-413.
4. Russell, M.W., L.A. Bergmeier, E.D. Zanders and T. Lehner. 1980. Infect. Immun. 28:486-493.
5. Russell, M.W., L.A. Bergmeier, E.D. Zanders and T. Lehner. 1980. Infect. Immun. 29:999-1006.
6. Smith, R., T. Lehner and P.C.L. Beverley. 1984. Infect. Immun. 46:168-175.
7. Zanders E.D., and T. Lehner. 1981. J. Gen. Microbiol. 122:217-225.

## Claims

1. An antibody or fragment thereof, which is able to combat dental caries, said antibody being obtainable by using as immunogen, Streptococcus sobrinus serotype d or streptococcal antigen thereof or a fragment of said antigen, said antigen fragment retaining the antigenic determinants characteristic of S. sobrinus serotype d, the antibody or fragment thereof cross reacting with S. mutans serotypes c, e, f and g.

2. A polyclonal antibody according to claim 1.

3. A monoclonal antibody according to claim 1.

4. A fragment of an antibody according to any one of the preceding claims, said fragment retaining sites that bind with both S. sobrinus serotype d and S. mutans serotype c.

5. An antibody or fragment thereof according to any one of the preceding claims of the IgG1 or IgG2b class.

6. A dental composition,suitable for topical application to the teeth, comprising an antibody or fragment thereof according to any one of the preceding claims together with a carrier or diluent.

7. A composition according to claim 6 in the form of a toothpaste, mouthwash, chewing gum, lozenge or gel.

8. An antibody or fragment thereof according to any one of claims 1 to 5 for use in a method of combatting dental caries in a mammal.

9. A composition according to claim 6 or 7 for use in a method of combatting dental caries in a mammal.

## Patentansprüche

1. Zur Bekämpfung von Zahnkaries fähiger Antikörper oder ein Fragment davon, dadurch **gekennzeichnet,**
daß der Antikörper unter Verwendung von Streptococcus sobrinus Serotyp d oder eines Streptokokken-Antigens davon oder eines Fragments des Antigens als Immunogen erhältlich ist, wobei das Antigenfragment die Eigenschaft der antigenen Determinanten von S. sobrinus Serotyp d beibehält und wobei der Antikörper oder ein Fragment davon mit den S. mutans Serotypen c, e, f und g kreuzreagiert.

2. Polyklonaler Antikörper nach Anspruch 1.

3. Monoklonaler Antikörper nach Anspruch 1.

4. Fragment eines Antikörpers nach einem der vorstehenden Ansprüche, wobei das Fragment die Stellen beibehält, die sowohl mit S. sobrinus Serotyp d als auch mit S. mutans Serotyp c binden.

5. Antikörper oder ein Fragment davon nach einem der vorstehenden Ansprüche aus der IgG1- oder IgG2b-Klasse.

6. Für die topische Anwendung an Zähnen geeignete Zusammensetzung, umfassend einen Antikörper oder ein Fragment davon nach einem der vorstehenden Ansprüche zusammen mit einem Träger oder Verdünnungsmittel.

7. Zusammensetzung nach Anspruch 6, formuliert als Zahnpasta, Mundwasser, Kaugummi, Tablette oder Gel.

8. Antikörper oder ein Fragment davon nach einem der Ansprüche 1 bis 5 zur Verwendung in einem Verfahren für die Bekämpfung von Zahnkaries bei einem Säuger.

9. Zusammensetzung nach Anspruch 6 oder 7 zur Verwendung in einem Verfahren für die Bekämpfung von Zahnkaries bei einem Säuger.

## Revendications

1. Anticorps ou fragment de celui-ci, apte à combattre la carie dentaire, ledit anticorps étant susceptible d'être obtenu en utilisant à titre d'immunogène, Streptococcus sobrinus sérotype d ou l'antigène streptococcique de celui-ci ou un fragment dudit antigène, ledit fragment d'antigène conservant les déterminants antigéniques caractéristiques de S. sobrinus sérotype d, l'anticorps ou fragment de celui-ci présentant une réaction croisée avec S. mutans sérotypes c, e, f et g.

2. Anticorps polyclonal selon la revendication 1.

3. Anticorps monoclonal selon la revendication 1.

4. Fragment d'un anticorps selon l'une quelconque des revendications précédentes, ledit fragment conservant les sites qui se lient à la fois avec S. sobrinus sérotype d et S. mutans sérotype c.

5. Anticorps ou fragment de celui-ci selon l'une quelconque des revendications précédentes, de la classe d'IgG1 ou d'IgG2b.

6. Composition dentaire, convenant à une application topique sur les dents, comprenant un anticorps ou fragment de celui-ci selon l'une quelconque des revendications précédentes, conjointement avec un support ou un diluant.

7. Composition selon la revendication 6, sous forme de dentifrice, de bains de bouche, de chewing-gum, de pastille ou de gel.

8. Anticorps ou fragment de celui-ci selon l'une quelconque des revendications 1 à 5, destiné à être utilisé dans un procédé pour combattre la carie dentaire chez un mammifère.

9. Composition selon la revendication 6 ou 7, destinée à être utilisée dans un procédé pour combattre la carie dentaire chez un mammifère.
